Europäisches Patentamt

European Patent Office

Office européen des brevets

(11) Veröffentlichungsnummer: **0 245 768**
**B1**

(12) **EUROPÄISCHE PATENTSCHRIFT**

(45) Veröffentlichungstag der Patentschrift:
07.11.90

(51) Int. Cl.⁵: **G21K 1/04**

(21) Anmeldenummer: 87106581.9

(22) Anmeldetag: 07.05.87

(54) Konturenkollimator für die Strahlentherapie.

(30) Priorität: 14.05.86 DE 3616141
03.04.87 DE 3711245

(43) Veröffentlichungstag der Anmeldung:
19.11.87 Patentblatt 87/47

(45) Bekanntmachung des Hinweises auf die Patenterteilung:
07.11.90 Patentblatt 90/45

(84) Benannte Vertragsstaaten:
CH DE FR GB LI NL SE

(56) Entgegenhaltungen:
DE-A- 3 030 332
DE-B- 1 010 659
DE-C- 192 300
US-A- 4 463 266

(73) Patentinhaber: Siemens Aktiengesellschaft,
Wittelsbacherplatz 2, D-8000 München 2(DE)

(72) Erfinder: Pastyr, Otto, Mannheimer Weg 6,
D-6906 Leimen(DE)
Erfinder: Maier-Borst, Wolfgang, Dr., Schlüsselweg 9,
D-6901 Dossenheim(DE)

**Beschreibung**

Die Erfindung bezieht sich auf einen Konturenkollimator für die Strahlentherapie mit einer vorgegebenen Anzahl von gegeneinander verschieblich angeordneten Blendplatten. Ein derartiger Kollimator ist aus der Druckschrift: Acta Radiologica, Suppl. 242 (1965), 1–142 bekannt. Sie bezieht sich insbesondere auf einen Vielblattkollimator, der zur Begrenzung des Strahlungsfeldes einer ionisierenden Strahlung eingesetzt wird, und zwar bevorzugt zur Begrenzung des Strahlungsfeldes von Gammastrahlung bei einem Linearbeschleuniger.

Die heute in der onkologischen Strahlentherapie eingesetzten Bestrahlungsgeräte sind mit Strahlenfeldkollimatoren ausgerüstet, die lediglich die Einstellung rechteckig begrenzter Strahlenfelder zulassen. Es ist heute jedoch bekannt, daß bei vielen onkologischen Fragestellungen bessere Therapieergebnisse erreicht werden können, wenn die Strahlendosisverteilung der meist unregelmäßigen Form der Zielvolumina (Tumore sind meist nicht kugelförmig) angepaßt wird.

Hierzu werden in der Strahlentherapie unregelmäßig geformte Zusatzkollimatoren individuell angefertig. Die zur Herstellung solcher Kollimatoren erforderlichen Geräte sind kommerziell verfügbar. Es handelt sich dabei um Geräte, die es ermöglichen, anhand von Röntgenbildvorlagen irreguläre Strahlenfeldformen aus Hartschaumplatten auszuschneiden und diese mit Metallegierungen niedrigen Schmelzpunktes auszugießen. Diese Herstellungsprozedur ist nur für einzelne Einstrahlungswinkel durchführbar und recht aufwendig.

Bereits 1965 wurde von Takahashi ("Confirmation Radiotherapy", Acta Radiologica, Suppl. 242 (1965), 1 - 142) der Einsatz beliebig verstellbarer Kollimatoren nach dem Vielblattprinzip (sog. "Multi-Leaf-Kollimatoren") vorgeschlagen. Solche von Hand einstellbaren Kollimatoren wurden in der Strahlentherapie dann im Laufe der Zeit weltweit auch in verschiedenen Bestrahlungszentren eingesetzt. Der Vorteil gegenüber den nach dem Gußprinzip hergestellten Kollimatoren ist jedoch gering: An die Stelle des Ausschneidens und Gießens tritt die ebenfalls zeitaufwendige Handeinstellung der einzelnen "Kollimatorblätter" oder "Blendplatten".

Mit der Verfügbarkeit billiger mikroelektronischer Steuerkomponenten wird heute an verschiedenen Bestrahlungszentren die Entwicklung motorisch einstellbarer Vielblattkollimatoren vorangetrieben. Diese Kollimatoren sind für den Einsatz an Neutronenbestrahlungsanlagen, an Photonenstrahlungsquellen und insbesondere an Linearbeschleunigern vorgesehen. Die genannten Entwicklungen haben das Prinzip des Einzelblattantriebes gemeinsam: Jedes Blatt (Blendplatte) des Vielblattkollimators wird von einem eigenen Schrittmotor angetrieben. Die Zahl der benötigten Schrittmotoren ist gleich der Zahl der Einzelblätter. Der Aufwand an komplizierter und störungsanfälliger Elektronik und der Platzbedarf zum Einbau eines solchen Kollimators in ein Bestrahlungsgerät ist sehr hoch, da insgesamt mindestens 40 Blätter und dadurch 40 Schrittmotoren benötigt werden.

Aus der DE-C 192 300 ist bereits ein Konturenkollimator bekannt, bei dem zur Ausblendung eines vorgegebenen Profils aus dem Strahlungsfeld einer Röntgenstrahlungsquelle zwei gegenüberliegend angeordnete Gruppen von gegeneinander verschieblichen, für Röntgenlicht undurchlässigen Stäbchen vorgesehen sind. Dieser Kollimator ist nicht für die Strahlentherapie, bei der insbesondere hochenergetische Photonen (Gammastrahlung) zur Anwendung kommen, geeignet, da keine eigentlichen "Blendplatten" verwendet werden. Darüber hinaus ist lediglich eine manuelle Einstellung der Stäbchen vorgesehen. Eine solche manuelle Einstellung ist jedoch in der Regel zu langsam für die Strahlentherapie, wo nacheinander mehrere Strahlungsfelder mit unterschiedlichen Profilen zur Anwendung kommen.

Aus der DE-AS 1 010 659 ist ein Kollimator zur Ausblendung eines Nutzstrahlenbündels aus der Strahlung eines Strahlers hoher Energie, z. B. eines Kobalt-60-Präparates, bekannt, der Blendplatten aufweist, welche senkrecht zum Zentralstrahl des auszublendenden Bündels verstellbar sind. Bei diesem Kollimator ist je ein Verstellglied für jede einzelne Blendplatte vorgesehen. Ein allen Verstellgliedern gemeinsames Antriebsorgan, z. B. eine Antriebswelle, ist mit jedem der Verstellglieder lediglich über Reibkupplungen verbunden. Die Begrenzung des gewünschten Strahlungsfeldes ist durch eine Lochplatte vorgegeben, in die Stifte hineingesteckt werden. Bei einem solchen Kollimator ist es ersichtlich schwierig, innerhalb kurzer Zeit ein neues Bestrahlungsfeld einzustellen. Darüber hinaus ist der Kollimator für Pendelungen in einer Vertikalebene, die z.B. bei der Therapie mit einem Linearbeschleuniger durchgeführt werden, nicht geeignet. Bei einer gewissen Stellung spricht nämlich unter der Wirkung des Gewichts der Kollimatorplatten die Rutschkupplung an; damit würden Blendplatten herausfallen, wodurch sich eine Verstellung der Kontur ergibt. Darüber hinaus würde eine Rutschkupplung nicht die Patientensicherheit bei der sogenannten Einzeit-Bestrahlung, bei der die gesamte erforderliche Dosis in einer Fraktion abgegeben wird, gewährleisten.

Aus der DE-A 30 30 332 ist eine Primärstrahlenblende für ein Röntgenuntersuchungsgerät bekannt, bei dem mehrere, den Strahlenkegel von verschiedenen Seiten begrenzende Einblendelemente aus paketweise zusammengefaßten dünnen, aneinanderliegenden, in Längsrichtung gegeneinander verschiebbaren Metallstreifen verwendet werden. Zur Einstellung durch Fernsteuerung trägt jeder Metallstreifen an seiner der Symmetrieachse der Primärstrahlenblende abgewandten Seite eine sich quer zur Verschieberichtung und senkrecht zur Einblendebene erstreckende Nase. Darüber hinaus ist jedem Metallstreifenpaket ein mit den einzelnen Metallstreifen in Eingriff bringbares, von einem x-, y-Antrieb verstellbares Verstellglied zugeordnet. Auch dieser Konturenkollimator ist für geringe Energien vorgesehen, da relativ kurze Blendplatten verwendet werden. Bei einer 360°-Drehung des Kollimators um einen Patienten würden die einzelnen Blendplatten herausfallen, weil keine Verriegelung vorgesehen ist. Zwar ist ein Einstellorgan für die einzelnen Blendplatten vorhanden, aber dies kann nur geringe Blendplattengewichte bewegen. Darüber hinaus ist es infolge seiner Konstruktion darauf beschränkt, nur weichgeformte Konturen oder Profile, also solche ohne Stufen, einzustellen.

Aufgabe der vorliegenden Erfindung ist es, einen Konturenkollimator der eingangs genannten Art so auszugestalten, daß eine einfache, feinstufige Verstellbarkeit der Blendplatten mit geringem Aufwand gewährleistet ist, wobei gleichzeitig eine ausreichende Sicherheit gegen die Verstellung einer gewählten Bestrahlungskontur gegeben sein soll.

Diese Aufgabe wird erfindungsgemäß gelöst durch

a) eine Verzahnung an jeder Blendplatte,

b) ein der vorgegebenen Anzahl von Blendplatten gemeinsames Verstellorgan zum Verschieben jeweils einer ersten der Blendplatten gegenüber den restlichen Blendplatten, welches Verstellorgan mit der Verzahnung der ersten Blendplatte im Eingriff steht,

c) eine mit den Verzahnungen der restlichen Blendplatten im Eingriff stehende Verriegelungseinrichtung und

d) eine Einrichtung zum Verschieben des Verstellorgans von der Verzahnung der ersten Blendplatte zur Verzahnung einer weiteren zweiten Blendplatte, bei welchem Verschieben die erste Blendplatte verriegelt und die zweite Blendplatte entriegelt wird.

Die zweite Blendplatte braucht dabei nicht die unmittelbar neben der ersten Blendplatte gelegene Platte zu sein; es kann sich auch um eine weitere Blendplatte handeln.

Bei einem solchen Konturenkollimator ist gewährleistet, daß sich die für eine vorgegebene Bestrahlungsrichtung eingestellte Kontur nicht selbsttätig verändert. Dadurch ist der Kollimator besonders geeignet für die Anwendung in Verbindung mit Strahlungsquellen, die sich während der Bestrahlung bewegen. Insbesondere läßt sich dieser Kollimator beim Umkreisen eines zu bestrahlenden Tumors einsetzen. Tumore sind normalerweise unregelmäßig geformt. In der Bestrahlungstherapie werden sie in der Regel aus verschiedenen Bestrahlungsrichtungen angegangen. Die Durchlaßöffnung oder Kontur des Konturenkollimators läßt sich also beim kontinuierlichen oder schrittweisen Umkreisen des Tumors jeweils an dessen aktuelle, d. h. aus der Bestrahlungsrichtung gesehene Kontur schnell anpassen. Dadurch werden kurze Bestrahlungszeiten ermöglicht, was insbesondere für hochenergetische Gammastrahlung, die von einem Linearbeschleuniger abgegeben wird, von Bedeutung ist. Bei bekanntem Profil des Tumors, das beispielsweise durch eine Computer-Tomographie-Aufnahme und darauf folgende dreidimensionale Berechnung und Bestrahlungsplanung ermittelt sein kann, läßt sich beim Umkreisen die Kontur motorisch verstellen, ohne daß dabei befürchtet zu werden braucht, daß einzelne Blendplatten ihre vorgegebene Position verändern oder gar herausfallen. Dadurch wird erreicht, daß der Tumor eng umgrenzt bestrahlt und gesundes Gewebe optimal geschont wird.

Eine Ausgestaltung der Erfindung zeichnet sich dadurch aus, daß eine weitere vorgegebene Anzahl von Blendplatten neben den erstgenannten Blendplatten angeordnet ist, und daß diesen weiteren Blendplatten eine gleichartig aufgebaute Verriegelungs- und Verschiebeeinrichtung zugeordnet ist. Auf diese Weise läßt sich von zwei Seiten oder Stellen aus eine Verschiebung von Blendplatten zwecks Einstellung einer neuen Kontur durchführen, was die Zugriffszeit und damit die Bestrahlungsdauer verkürzt.

Eine weitere bevorzugte Ausgestaltung zeichnet sich durch einen symmetrischen Aufbau bezüglich einer Mittellinie aus. Auf beiden Seiten dieser Mittellinie sind also jeweils gegeneinander verschiebliche Blendplattenpakete angeordnet. Die Blendplatten der beiden Pakete sind dabei aufeinanderzu beweglich angeordnet. Bevorzugt ist die Anordnung so getroffen, daß die Blendplatten jeweils über die Mittellinie hinaus in den Bereich des anderen Blendplattenpakets hineingeschwenkt werden können. Auf diese Weise lassen sich auch asymmetrische Bestrahlungsfelder einstellen.

Bei geeigneter Wahl der Zahnabstände in der Verzahnung jeder Blendplatte können die einzelnen Blendplatten mehr oder weniger weit feinstufig in Richtung auf die Mittellinie verschoben werden. Auf diese Weise läßt sich ein gewünschtes Bestrahlungsprofil mit großer Präzision einstellen.

Es hat sich nun erwiesen, daß man einerseits für die Blendplatten ein strahlungsresistentes und strahlungsundurchlässiges Material verwenden muß, und daß man andererseits ein Material wählen sollte, das aus Kostengründen mit den üblichen Arbeitsmethoden zu bearbeiten ist. Beispielsweise soll es möglich sein, in den Blendplatten ohne allzu großen Aufwand Führungsrillen und Verzahnungen anzubringen oder die Blendplatten keilförmig zu schleifen. Ein Material, das den genannten Anforderungen gerecht wird, ist beispielsweise eine Wolfram-Nickel-Legierung. Eine solche Legierung ist ein relativ hartes, nicht-elastisches Material. Werden Blendplatten aus einem solchen Material, also weitgehend starre Platten, von der Mittellinie nach außen verschoben (bei feststehenden Außenplatten), d. h. aus dem Zentrum der Strahlung heraus, dann können aus geometrischen Gründen die Blendplatten klemmen, was ein Zurückfahren schwierig macht. Von Bedeutung ist dieses Problem dann, wenn eine Anzahl an Blendplatten (z.B. 27), die sich in Richtung auf den Ursprungsort der Strahlung verjüngen, verwendet wird.

Es stellt sich somit die Aufgabe, den Konturenkollimator derart weiterzubilden, daß auch bei Einsatz von Blendplatten aus einem relativ harten Material beim Verschieben kein Klemmen auftritt.

Diese Aufgabe wird dadurch gelöst, daß die Innenflächen der Außenplatten unter vorgegebenem Winkel zur Normalen der Mittellinie ausgerichtet sind, und daß die Blendplatten an der der Mittellinie der Strahlung zugewandten Kante eine geringere Dicke aufweisen als an der von der Mittellinie der Strahlung abgewandten Kante.

Weitere vorteilhafte Ausführungsformen sind in den Unteransprüchen gekennzeichnet.

Im folgenden werden Ausführungsbeispiele der Erfindung anhand von zwölf Figuren näher erläutert. Es zeigen:

Fig. 1 einen Linearbeschleuniger, bei dem ein Konturenkollimator nach der Erfindung eingesetzt wird;

Fig. 2 einen seitlichen Blick auf einen Konturenkollimator nach der Erfindung;

Fig. 3 einen Blick auf eine Blendplatte, die im rechten Blendplattenpaket nach Fig. 2 eingesetzt wird;

Fig. 4 einen Blick auf die das Strahlungsbündel definierende gerade Kante der Blendplatte nach Fig. 3;

Fig. 5 einen Blick (Ansicht A) auf den Konturenkollimator nach Fig. 2, teilweise aufgebrochen;

Fig. 6 einen Blick (Ansicht B) auf den linken Teil des Konturenkollimators nach Fig. 2 ohne Blendplatten;

Fig. 7 einen Schnitt (Schnitt C-C) durch den rechten Teil des Konturenkollimators nach Fig. 2;

Fig. 8 einen Blick von oben auf einen Konturenkollimator mit keilförmigen Außenplatten entsprechend Fig. 5;

Fig. 9 einen Blick auf eine Blendplatte, die in der rechten vorderen Blendplatten-Paketgruppe nach Fig. 8 eingesetzt wird;

Fig. 10 einen Schnitt durch den linken Teil der Blendplatte nach Fig. 9 entlang der Linie X-X;

Fig. 11 einen Schnitt durch den rechten Teil der Blendplatte nach Fig. 9 entlang der Linie XI-XI; und

Fig. 12 einen Blick von oben auf die Blendplatte nach Fig. 9.

Im folgenden werden für gleiche Bauteile dieselben Bezugszeichen verwendet.

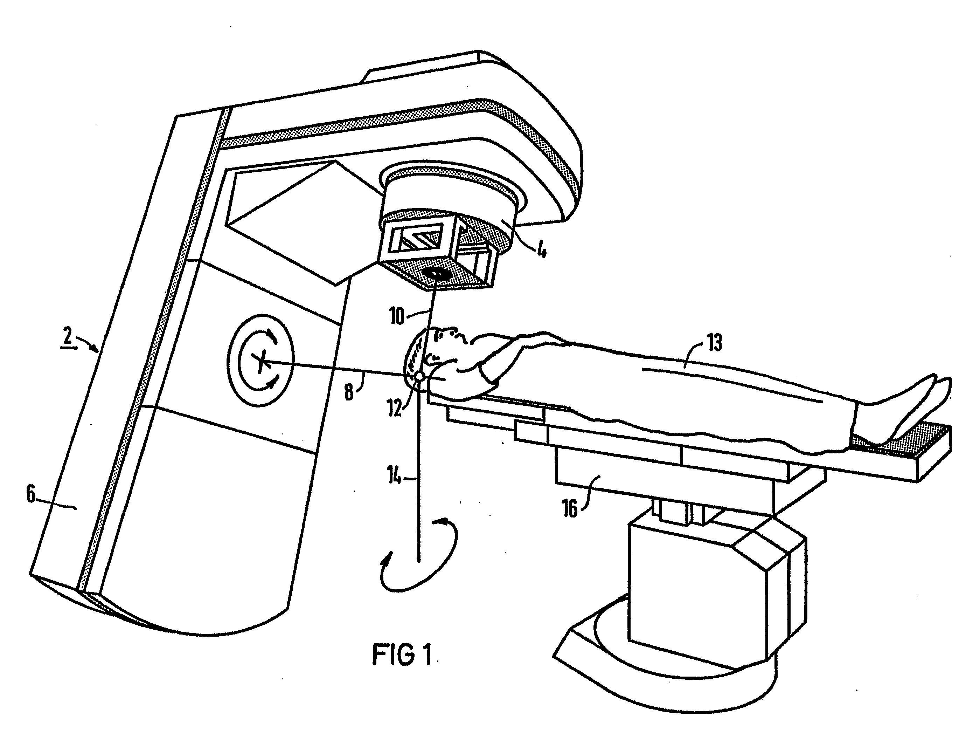

In Fig. 1 ist ein Teil eines Linearbeschleunigers 2 bekannter Ausführung dargestellt, bei dem ein Konturenkollimator 4 mit serieller Ansteuerung der einzelnen Blendplatten eingesetzt ist. Der Linearbeschleuniger 2 umfaßt eine Gantry 6 (Gerüst), die den Konturenkollimator 4 enthält und die um eine horizontale Rotationsachse 8 im Laufe einer therapeutischen Behandlung gedreht wird. Der Hauptstrahl des aus dem Linearbeschleuniger 2 austretenden Strahlungsbündels ist mit 10 bezeichnet. Er ist während der Behandlung auf die zu behandelnde Zone 12 eines Patienten 13 gerichtet, die im Isozentrum liegt. Die Rotationsachse 8 der Gantry 6, die Rotationsachse 14 eines Behandlungstisches 16 und die Strahlachse 10 schneiden sich im Isozentrum.

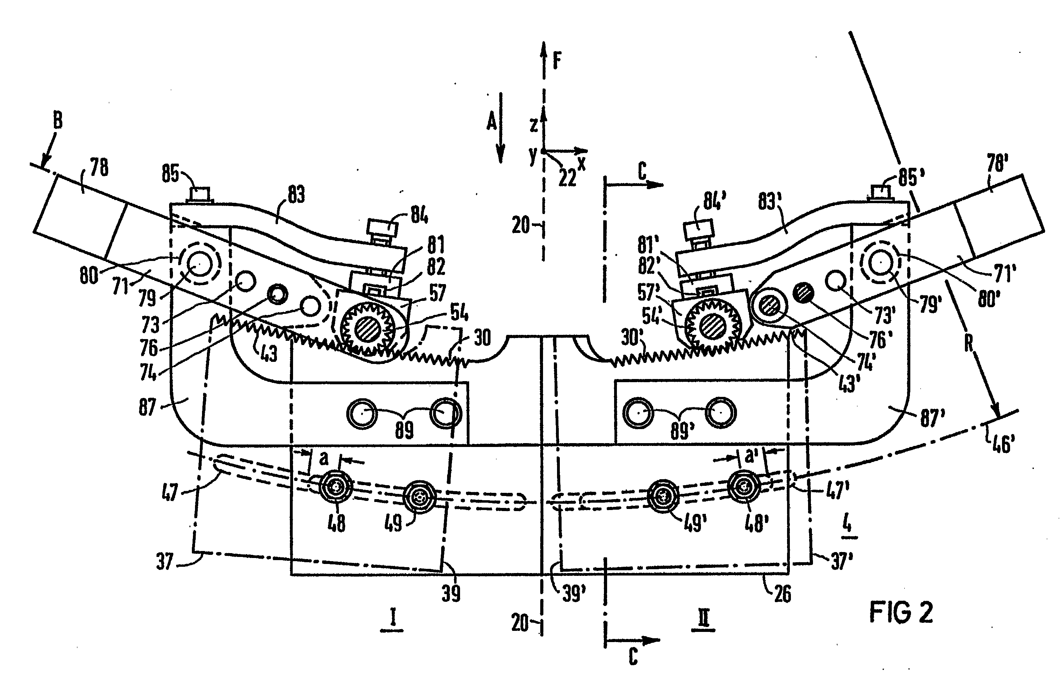

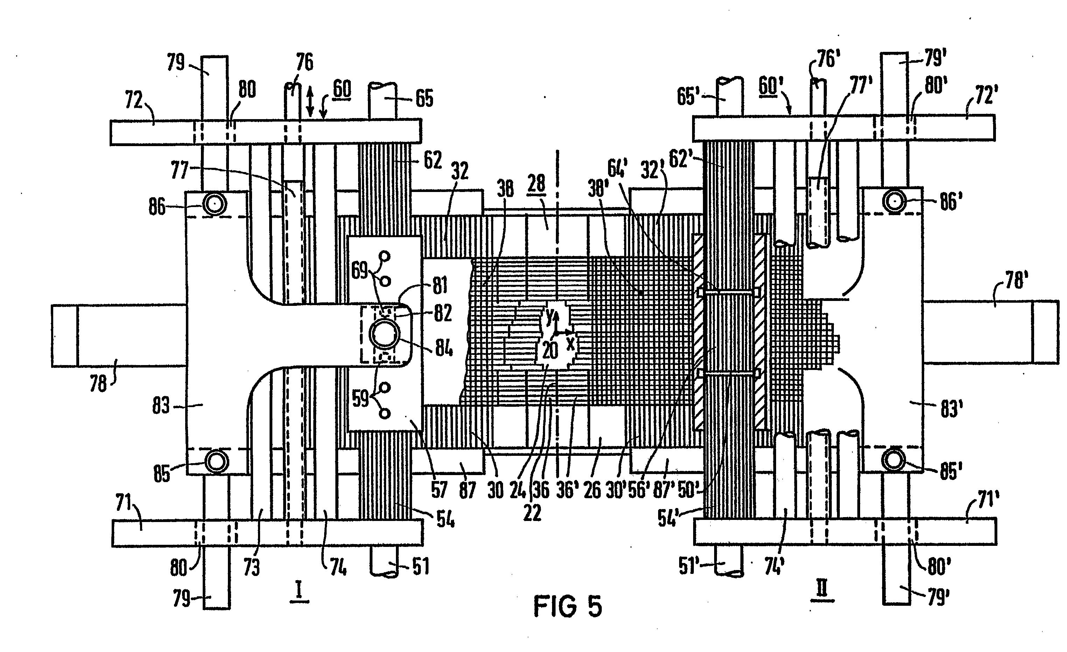

In Fig. 2 sind in einer seitlichen Darstellung Einzelheiten des Konturenkollimators 4 gezeigt; für die Beschreibung ist die vertikale Bestrahlungsstellung angenommen. Dieser Kollimator 4 besteht aus zwei Teilen oder Seiten I und II, die bezüglich einer vertikalen Ebene, durch die eine Symmetrielinie 20 verläuft, symmetrisch zueinander ausgebildet sind. Die Symmetrielinie 20 fällt bei optimaler Justierung mit der Hauptstrahlrichtung des von einem Fokus F ausgehenden Strahlungsbündels hochenergetischer Strahlung zusammen. Bei dieser Strahlung kann es sich insbesondere um Röntgenstrahlung handeln. Wie aus Fig. 5 (Blick in Richtung A in Fig. 2) hervorgeht, liegt zwischen den Seiten I und II eine Mittellinie 22, die zusammen mit der Symmetrielinie 20 die vertikale Symmetrieebene 20, 22 aufspannt. Die Mittellinie 22 fällt mit der y-Achse eines x-, y-, z-Koordinatensystems zusammen. Dessen z-Achse wird durch die Symmetrielinie 20 gebildet. Die im Bereich der Symmetrielinie 20 liegende, durch Ausblendung erzielte Strahlenkontur (Profil) ist mit 24 bezeichnet.

Aus einer Betrachtung der Fig. 2 und 5 ergibt sich, daß in einigem Abstand von einander eine erste und eine zweite vertikal gestellte, seitliche Außenplatte 26 bzw. 28 parallel zueinander angeordnet sind. Diese Außenplatten 26, 28 sind an ihren Oberkanten ab einem gewissen Abstand zur Symmetrieebene 20, 22 in kreisbogenförmigen Ausnehmungen oder Rändern mit Verzahnungen 30, 30' bzw. 32, 32' versehen.

Die Seiten I und II sind bezüglich der Symmetrieebene 20, 22 symmetrisch aufgebaut, so daß es genügt, lediglich die linke Seite I im einzelnen zu beschreiben. Die entsprechenden, auf der rechten Seite II gelegenen Bauelemente sind jeweils mit einem Strich an der zugeordneten Bezugsziffer versehen. Sie haben den gleichen Aufbau und dieselbe Funktion. Auf sie wird im folgenden auch gelegentlich eingegangen.

Zwischen den beiden seitlichen Außenplatten 26, 28 ist ein Paket aus zwei Gruppen von gegeneinander verschieblichen Blendplatten 36, 38 angeordnet. Alle Blendplatten 36, 38 in der vorderen bzw. hinteren Gruppe sind in gleicher Weise aufgebaut und nebeneinander liegend angeordnet. Sie sind dennoch mit verschiedenen Bezugszeichen belegt, weil sie - wie im folgenden näher geschildert wird - durch verschiedene Einrichtungen betätigt werden. Das gesamte Paket kann beispielsweise 28 Blendplatten 36, 38 umfassen. Dasselbe gilt für die Blendplatten 36', 38' des rechts angeordneten Blendplattenpakets.

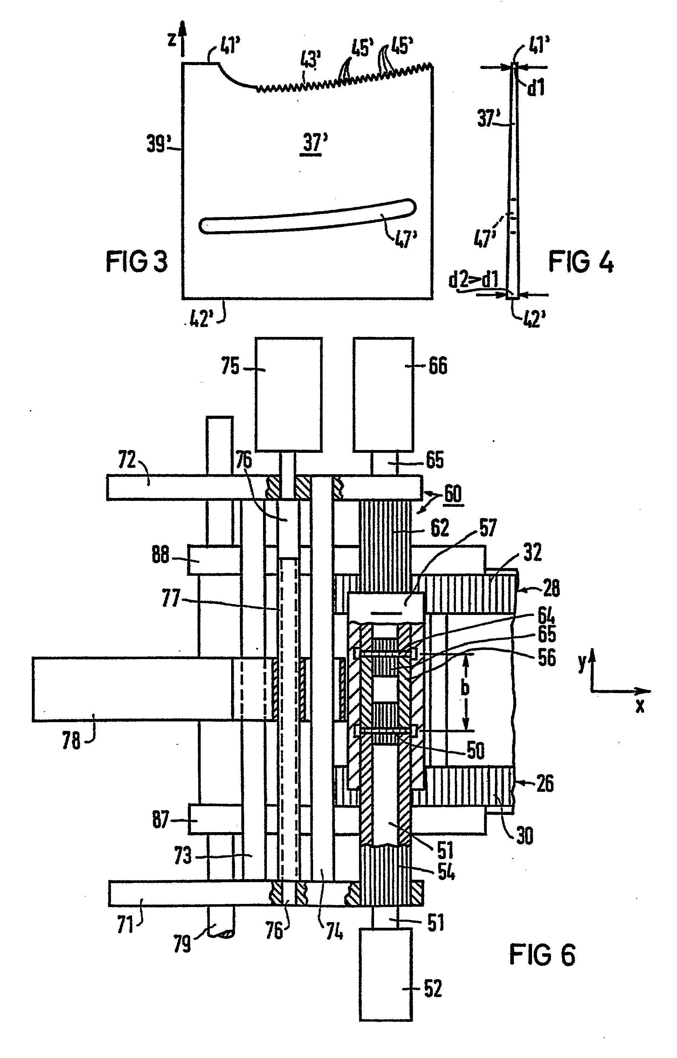

In den Fig. 3 und 4 ist eine beliebige der identischen Blendplatten 36', 38' der rechten Seite II näher dargestellt und mit 37' bezeichnet. Sie ist im wesentlichen quadratisch geformt. Die links gelegene gerade Kante 39' dient zur Begrenzung des Strahlungsbündels. Die obere Kante 41' ist mit einer kreisbogenförmigen Ausnehmung (Rand) versehen, in die rechts eine Verzahnung 43' eingelassen ist. Diese Verzahnung 43' besitzt vorzugsweise dreieckförmige Zähne 45', die in einem Abstand von jeweils etwa 1,5 mm angeordnet sind. Infolge dieser feinen Verzahnung 43' können die Blendplatten 36', 38' individuell und feinstufig etwa parallel zur x-z-Ebene geschwenkt werden, und zwar jeweils in Schritten von 1,5 mm. Die Verzahnung 43' stimmt mit den rechten Verzahnungen 30', 32' der festgehaltenen Außenplatten 26, 28 überein.

Unterhalb ihrer Mitte ist in die Blendplatte 37' eine bogenförmige Führungsrille 47' eingelassen. Diese gekrümmte Führungsrille 47' dient zur Führung der Blendplatte 37' derart, daß die gerade Kante 39' immer parallel zum äußeren Strahl des eingegrenzten Strahlenkegels verläuft. Mit anderen Worten, während der Führung entlang der Führungsrille 47' ist die gerade Kante 39' immer auf den Fokus F der Strahlungsquelle ausgerichtet. Der Krümmungsradius R für diese Führungsrille 47' kann beispielsweise 53 cm betragen. Der Krümmungsradius R ist in Fig. 2 am Schwenkbogen 46' eingezeichnet. Die Blendplatte 37' kann insbesondere aus Wolfram oder einem Wolfram enthaltenden Material bestehen. Nach Fig. 4 besitzt sie einen keilförmigen Querschnitt. Mit anderen Worten, die der Strahlungsquelle und damit dem Fokus F zugewandte Kante 41' besitzt eine Dicke d1, die kleiner ist als die Dicke d2 an der der Strahlungsquelle abgewandten Kante 42'.

Die Blendplatten 36, 38 der linken Seite I sind identisch ausgebildet zu den Blendplatten 36', 38'. Sie sind nur seitenverkehrt zwischen den Außenplatten 26, 28 angeordnet. Generell werden sie jeweils als Blendplatte 37 bezeichnet.

In Fig. 2 ist je eine Blendplatte 37, 37' aus dem Paket der linken bzw. rechten Seite I bzw. II gestrichelt eingezeichnet. Beide Blendplatten 37, 37' sind in einer von der Symmetrieebene 20, 22 herausgeschobenen Position gezeigt. Sie sind gegenüber den Außenplatten 26, 28 verschoben. In Fig. 2 ist auch zu sehen, daß die beiden Blendplatten 37, 37' in dieser Position um den Fokus F herumgeschwenkt sind. Zum Führen entlang der Führungsrillen 47, 47' beim Schwenken (Antrieb über die Verzahnungen 43, 43') dienen Führungsbolzen 48, 49 bzw. 48', 49', die die seitlichen Außenplatten 26, 28 miteinander verbinden. Wenn der Konturenkollimator 4 in einer symmetrischen Stellung und ganz geschlossen ist, dann liegen die geraden Kanten 39, 39', die auf den Fokus F ausgerichtet sind, in der Symmetrieebene 20, 22. Damit aber die Blendplatte 37 auch in den Bereich der rechten Seite II hineinfahren kann (und entsprechend die Blendplatte 37' in den Bereich der linken Seite I), sind die Führungsrillen 47, 47' etwas länger ausgeführt als für die genannte symmetrische Schließstellung eigentlich erforderlich. Dies ist durch den Abstand a bzw. a' in Fig. 2 angedeutet. Es hat sich gezeigt, daß man mit einem Abstand a = a' = 10 mm bei einer Blendplatte 37, 37' von einer Basisbreite von 10 cm ausreichend weit in die benachbarte Seite II bzw. I hineinfahren kann.

Für die vorgegebene Anzahl von Blendplatten 36 der vorderen Gruppe ist ein gemeinsames Verstellorgan vorgesehen. Dieses dient zum seriellen Verschieben jeweils einer ausgewählten ersten dieser Blendplatten 36 gegenüber den restlichen Blendplatten 36. Wie weiter unten erläutert ist, steht dieses Verstellorgan mit der Verzahnung 43 der ausgewählten Blendplatte 36 im Eingriff. Weiterhin ist eine mit den Verzahnungen 43 der restlichen Blendplatten 36 der vorderen Gruppe im Eingriff stehende Verriegelungseinrichtung vorgesehen. Darüber hinaus existiert auch eine Einrichtung zum Verschieben des Verstellorgans von der Verzahnung der ausgewählten Blendplatte zur Verzahnung einer benachbarten zweiten Blendplatte. Wenn das Verstellorgan von der ersten zur zweiten Blendplatte 36 verschoben wird, wird durch letztere Einrichtung die erste Blendplatte 36 verriegelt und die benachbarte zweite Blendplatte 36 entriegelt. Nun kann auch die zweite Blendplatte 36 gegenüber allen nunmehr arretierten Blendplatten 36 verschoben (verschwenkt) werden. Stattdessen kann auch zu einer dritten, vierten, etc. Blendplatte 36 übergegangen und diese verschwenkt werden.

Ein entsprechendes Verstellorgan und entsprechende Einrichtungen sind auch für die hintere Gruppe der Blendplatten 38 vorgesehen. Die beiden Einrichtungen zum Verschieben der Verstellorgane sind weitgehend identisch, d. h. durch dieselben Bauelemente gebildet.

Zunächst werden das Verstellorgan und die Verriegelungseinrichtung für die vordere Gruppe der Blendplatten 36 betrachtet; später wird dann zu der hinteren Gruppe übergegangen.

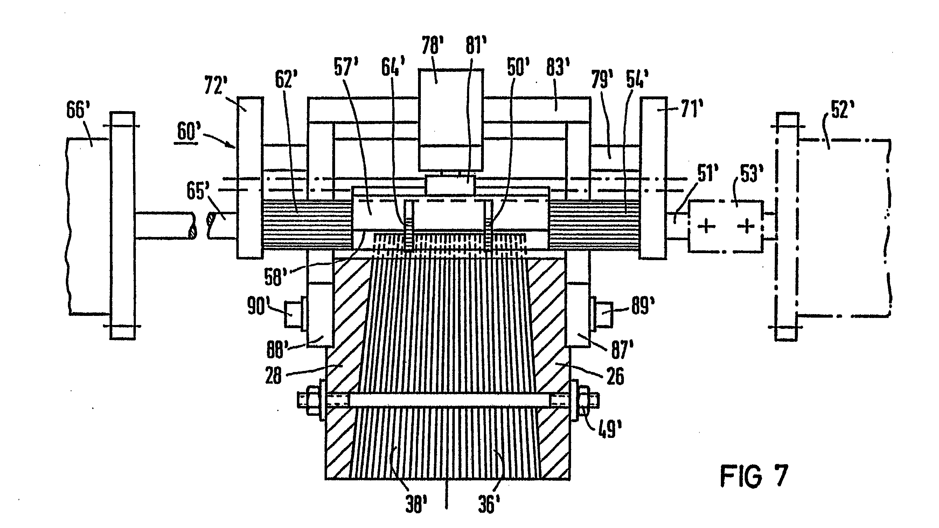

Nach Fig. 5 und 6 umfaßt dieses Verstellorgan ein Antriebszahnrad 50, das etwa die an der Verzahnung 43 gemessene Dicke d1 der zugeordneten Blendplatten 36 besitzt und mit der ausgewählten Blendplatte 36 im Eingriff steht. Dieses Zahnrad 50 ist über eine Einstellwelle 51 mit einem elektrischen Motor 52, insbesondere mit einem Schrittmotor, verbunden. Die Einstellwelle 51 reicht bis etwa zur Mitte des Systems, vergleiche Fig. 6. Wie aus Fig. 7 hervorgeht, kann zwischen dem vorderen Ende der Einstellwelle 51 und dem Motor 52 noch eine Kupplung 53 angeordnet sein, vgl. dort die Kupplung 53'. Der hintere Endbereich der Einstellwelle 51 ist verzahnt (vgl. Fig. 6) ausgebildet, und das Antriebszahnrad 50 ist auf diesen Endbereich aufgeschoben. Dreht sich der Motor 52, so dreht sich auch das Antriebszahnrad 50 in der gewünschen Richtung, wobei die ausgewählte Blendplatte 36 über ihre Verzahnung 43 mitgenommen wird, bis sie die gewünschte Endposition eingenommen hat.

Zu dem Verstellorgan 50, 51, 52 für die ausgewählte Blendplatte 36 gehört auch eine Verriegelungseinrichtung. Diese umfaßt eine erste und eine zweite verzahnte Welle 54 bzw. 56. Diese beiden Wellen 54, 56 sind breite Zahnräder mit Längsbohrungen, die axial zueinander ausgerichtet sind. Die Verzahnung ist so gewählt, daß sie mit den Verzahnungen 30, 32 der Seitenplatten 26, 28 und den Verzahnungen 43 der Blendplatten 36, 38 übereinstimmt. Das Antriebszahnrad 50 liegt axial zwischen den beiden Wellen 54 und 56. Sein Außendurchmesser sowie seine Verzahnung sind identisch mit dem Außendurchmesser bzw. der Verzahnung der Wellen 54, 56. Die Einstellwelle 51 ist dabei durch die Längsbohrung der Welle 54 hindurch- und in die Längsbohrung der Welle 56 hineingeführt. Die beiden Wellen 54, 56 sind durch ein hülsenförmiges Verbindungsteil 57 mit unterer Ausnehmung 58 (vgl. Fig. 7, Ausnehmung 58') zum Ermöglichen eines Eingriffs in die Verzahnungen 43 miteinander verbunden. Die hierfür verwendeten, oben angeordneten Befestigungsschrauben sind in Fig. 5 mit 59 bezeichnet. Die beiden Walzen oder Wellen 54, 56 sind Teil eines Rahmengestells 60 und somit nicht um ihre Längsachsen drehbar. Von dem gesamten Abschnitt zwischen dem vorderen Ende der Welle 54 und dem hinteren Ende der Welle 56 ist also nur derjenige Teil um die Längsachse drehbar, der durch das Antriebszahnrad 50 eingenommen wird. Die beiden Wellen 54, 56 dienen somit zur Arretierung der darunterliegenden Blendplatten 36, während das Antriebszahnrad 50 zum Schwenken, d. h. zur Verschiebung der ausgewählten Blendplatte 36 in x-Richtung, vorgesehen ist.

Aus Fig. 5 und 6 ist ersichtlich, daß noch eine dritte verzahnte Welle 62 gleichen Durchmessers und gleicher Verzahnung vorgesehen ist. Diese ist axial zu den beiden anderen Wellen 54, 56 ausgerichtet und hat dieselbe Breite wie die Welle 54. Sie dient mit der Welle 56 als Arretierorgan für die nicht ausgewählten Blendplatten 38 der hinteren Gruppe. Entsprechend ist zwischen den beiden Wellen 56 und 62 ein zweites Antriebszahnrad 64 gleichen Durchmessers angeordnet. Dieses sitzt auf dem einen Endbereich einer zweiten Einstellwelle 65, die dort ebenfalls eine Verzahnung aufweist. Die zweite Einstellwelle 65 ist am anderen Ende ebenfalls mit einem Motor 66 verbunden. Zwischen der zweiten Einstellwelle 65 und dem Motor 66 kann auch hier wieder eine (nicht gezeigte) Kupplung vorgesehen sein. Der Motor 66 ist zum Schwenken, d. h. zum Vorschub, für die jeweils ausgewählte hintere Blendplatte 38 parallel zur x-Richtung vorgesehen. Das motorische Verschwenken erfolgt solange, bis die betreffende Blendplatte 38 die gewünschte Endposition eingenommen hat. Die Bauteile 64, 65, 66 bilden somit gemeinsam ein teres Verstellorgan.

Durch Verwendung zweier Verstellorgane 52, 51, 50 sowie 66, 65, 64 ist gewährleistet, daß die Einstellzeit, in der die Blendplatten 36, 38 in der Verschieberichtung x eingestellt werden, halbiert wird.

Der Abstand b zwischen den beiden Antriebszahnrädern 50, 64 sollte bei kleinen zu bestrahlenden Feldern relativ klein gewählt werden. Dann können beide Antriebseinheiten zum Einsatz kommen, und es ergibt sich die erwähnte halbe Einstellzeit.

Das hülsenförmige Verbindungsstück 57 hält auch die dritte Welle 62 axial zu den beiden übrigen Wellen 54, 56 ausgerichtet. Zur Befestigung sind auch hier oben Schrauben 69 vorgesehen. Diese sind mit den Schrauben 59 in einer Linie parallel zur y-Achse angeordnet. Aus Fig. 6 ist ersichtlich, daß sämtliche drei Wellen 54, 56 und 62 Bestandteil des zuvor erwähnten Rahmengestells 60 sind.

Dieses Rahmengestell 60 ist Bestandteil der bereits erwähnten Einrichtung zum Verschieben des genannten Verstellorgans. Es umfaßt einen ersten und einen zweiten Seitenarm 71 bzw. 72, die parallel zur x-Achse ausgerichtet sind und etwa im mittleren Bereich durch zwei parallele Führungsstangen 73 bzw. 74 starr miteinander verbunden sind. Die beiden Führungsstangen 73, 74 sind dabei endseitig an den Seitenarmen 71, 72 befestigt. Zum Rahmengestell 60 gehört weiterhin die axiale Anordnung der Wellen 54, 56, 62. Dabei sind die verzahnten Wellen 54 und 62 endseitig fest mit den Seitenarmen 71 bzw. 72 verbunden. Dies kann dadurch erfolgen, daß die endseitige Verzahnung jeweils fest in ein Loch in den Seitenarmen 71 bzw. 72 eingepaßt ist. Dieses Rahmengestell 60 ist durch einen weiteren Schrittmotor 75 quer zu den Blendplatten 36, 38, d. h. in y-Richtung, verschiebbar. Der Motor 75 für die Querverschiebung ist in Fig. 6 neben dem Motor 66 gezeigt. Er ist dabei mit einer Einstellspindel 76, also mit einer Stange mit Gewinde 77, verbunden.

Die Einstellspindel 76 ist durch ein Loch im zweiten Seitenarm 72 geführt und im ersten Seitenarm 71 drehbar gelagert. Sie dreht sich in einem Gewinde, das in einem Halteblock 78 quer zur Längsrichtung angebracht ist. Der Halteblock 78 erstreckt sich dabei mit seiner Längsrichtung parallel zur xz-Ebene. Aus Fig. 2, 5 und 6 ist die parallele Anordnung der Einstellspindel 76 zwischen den Führungsstangen 73 und 74 ersichtlich. Der Halteblock 78 ist endseitig abgerundet. Er nimmt ebenfalls die Führungsstangen 73, 74, ggf. in je einem Linear-Kugellager (vgl. Fig. 6), auf.

Der Halteblock 78 ist von einer Gleitachse 79 durchsetzt, und zwar parallel zur y-Richtung. Die Gleitachse 79 ist dabei durch eine (nicht gezeigte) Schraube am Halteblock 78 befestigt. Sie ist beidseitig durch Löcher in den Seitenarmen 71, 72 hindurchgeführt und kann in diesen gleiten oder rutschen (bei Betätigung der Spindel 76). Dreht sich die Einstellspindel 76 unter der Wirkung des Motors 75 in einer der beiden Richtungen, so wird das gesamte in sich starre Rahmengestell 60, bestehend aus den Bauteilen 54, 56, 57, 62, 71, 72, 73, 74 mit den Teilen 50, 51, 52 und 64, 65, 66 in einer Richtung parallel zur y-Achse verschoben. Der Halteblock 78 bleibt dabei stehen. Die Verschiebung erfolgt jeweils in ganzen Schritten der Blendplattenstärke d1, z. B. jeweils in ganzzahligen Vielfachen von d1 = 3 mm. Auf diese Weise wird die zu verstellende Blendplatte 36, 38 ausgewählt. Beim Verschieben werden übrigens beide Zahnräder 50, 64 gleichzeitig parallel zu sich selbst in derselben Richtung +y oder -y verschoben. Zur Verminderung der Reibung bei der Verschiebung in der Richtung parallel zur y-Achse sind im Rahmengestell 60 Linearkugellager 80 eingebaut, durch die die Achse 79 gleitet.

Die drei verzahnten Wellen 54, 56, 62 samt Antriebszahnrädern 50, 64 werden durch eine Druckeinrichtung an die Verzahnungen 43 sowie 30, 32 der Blendplatten 36, 38 bzw. der Außenplatten 26, 28 angedrückt. Diese Druckeinrichtung umfaßt ein Andruckstück 81, das beim Verschieben des Rahmengestells 60 in y-Richtung auf der oberen Fläche des Verbindungsteils 57 gleitet. Damit es beim Gleiten an den Schrauben 59, 69 nicht hängenbleibt, ist es an seiner unteren Seite mit einem Querschlitz 82 versehen.

Die Druckeinrichtung weist weiterhin einen Andruckbügel 83 auf. Dieser ist T-förmig und geschwungen ausgebildet. An dessen Fuß ist eine Einstellschraube 84 vorgesehen, die auf das Andruckstück 81 drückt. Die Einstellschraube 84 dient zum Einstellen des Anpreßdrucks der Wellen 54, 56, 62 gegen die Verzahnungen 30, 32, 43. Das Gegenlager wird durch die Einspannfläche zwichen dem Querarm des T-förmigen Andruckbügels 83 und der End- oder Oberfläche zweier Seitenbügel 87, 88 im Bereich zweier Verschraubungen 85, 86 gebildet.

Der T-förmige Andruckbügel 82 ist durch die Verschraubungen 85, 86 an den beiden beabstandeten, parallel ausgerichteten Seitenbügeln 87 bzw. 88 befestigt. Die beiden Seitenbügel 87, 88 sind jeweils L-förmig ausgebildet und mittels zweier Schrauben 89 bzw. 90 an den Außenplatten 26 bzw.

28 befestigt. Die Seitenbügel 87, 88 schließen somit auch die Blendplatten 36, 38 zwischen sich ein. Damit Platz für die y-Verschiebung zur Verfügung steht, ist der Abstand zwischen den Seitenbügeln 85, 86 um einiges kleiner als der Abstand zwischen den Seitenarmen 71, 72.

Das Andruckstück 81 könnte auch durch ein anderes Bauteil mit weniger Reibung ersetzt werden.

Soll eine der Blendplatten 36, 38 in x-Richtung verschoben werden, so wird folgendermaßen vorgegangen: Zunächst wird der Antriebsmotor 75 für die Querverstellung betätigt. Die resultierende seitliche Verstellung des Rahmengestells 60 in y-Richtung erfolgt in ganzen Schritten der Blendplattenstärke d1, beispielsweise jeweils um 3 mm. Auf diese Weise wird die zu verstellende Blendplatte 36, 38 ausgewählt, und zwar durch eins der beiden Antriebszahnräder 50, 64. Ist eine Blendplatte 36, 38 ausgewählt, so wird die Verstellung in x-Richtung vorgenommen. Es muß dabei betont werden, daß nur die ausgewählte Blendplatte 36, 38, also nur diejenige, die sich gerade im Eingriff mit dem Antriebszahnrad 50, 64 befindet, verstellt werden kann. Alle anderen Blendplatten 36, 38 sind durch die drei Wellen 54, 56, 62 arretiert. Die Verstellung der betreffenden Blendplatte 36, 38 erfolgt entweder über die erste Einstellwelle 51 durch den ersten Motor 52 oder über die zweite Einstellwelle 65 durch den Antriebsmotor 66. Diese Einstellwellen 51, 65 werden in ganzzahligen Zahnschritten gedreht, bis die betreffende Blendplatte 36, 38 die zuvor ausgewählte Stellung im Strahlprofil 24 erreicht hat.

Es soll noch angemerkt werden, daß bevorzugt eine Einrichtung zur Rückmeldung der Verschiebestellung der einzelnen Blendplatten 36, 38 vorgesehen sein kann. Eine solche (nicht gezeigte) Einrichtung kann einen Zähler umfassen, der bei jedem Verschieben die Zahl der betätigten Zähne der Verzahnung 43 der betreffenden Blendplatte 36, 38 zählt. Dabei kann es sich um einen mechanischen oder optischen Zähler handeln. Es kann sich aber auch um einen Zähler handeln, der die Schritte des gerade betätigten Schrittmotors 52 bzw. 66 zählt. Die Anzahl der zurückgelegten Schritte, die durch elektrische Impulse charakterisiert sind, ist dabei ein Maß für die Positionierung in x-Richtung.

Es war bereits dargelegt worden, daß die Wellen 54, 56, 62 als Arretier- oder Bremselemente wirken, die alle - bis auf zwei - der Blendplatten 36, 38 festhalten. Stattdessen könnte auch ein anderes Halte- oder Arretierorgan gewählt werden, das in die Verzahnung 43 der Blendplatten 37 eingreift. Die Verzahnung 43 braucht dabei nicht unbedingt an einem Plattenrand zu liegen.

Nach Einstellen aller Blendplatten 36, 38 bleiben die Antriebszahnräder 50, 64 über den letzten zu verstellenden Blendplatten 36, 38 stehen. Diese werden durch den zugeordneten, unter Spannung stehenden Schrittmotor 52, 66 gehalten. Bei Ausfall der Antriebseinheit könnten sich nun die letzten Blendplatten, auf denen sich gerade die Antriebszahnräder befinden, aus ihrer Position bewegen. Dies wird vermieden, indem nach Einstellen der Blendplatten die Verriegelungseinrichtung 71 bis 76 noch um eine halbe Blendenplattenstärke weiterfährt. Damit sind alle Blendplatten 36, 38 mechanisch verriegelt.

Mittels des in den Fig. 2 bis 7 gezeigten Konturenkollimators läßt sich relativ rasch ein gewünschtes Strahlenprofil 24 einstellen. Auch beim Umkreisen des Konturenkollimators 4 während einer strahlentherapeutischen Behandlung muß nicht befürchtet werden, daß sich infolge ihres Gewichts einige der Blendplatten 36, 38, 36', 38' aus der vorgegebenen Positionierung lösen und damit die Strahlenkontur 24 verändern. Während einer solchen Umkreisung kann schrittweise in ausgewählten Umkreisungspositionen die Strahlenkontur neu eingestellt werden, um so ein optimales Strahlungsfeld zu applizieren.

Zusammenfassend läßt sich zu dem in den Figuren 2 bis 7 dargestellten Ausführungsbeispiel folgendes sagen: Zum Antrieb aller Blendplatten 36, 38 werden auf jeder Seite I, II des Konturenkollimators nur drei Schrittmotoren 52, 66, 75 benötigt, so daß zur Steuerung des gesamten Kollimatorsystems, unabhängig von der Blendplattenzahl, nur sechs Schrittmotoren 52, 66, 75 und 52', 66', 75' benötigt werden.

Der Unterschied zu anderen technischen Lösungen der Einzelblattsteuerung besteht vorliegend darin, daß die einzelnen Blendplatten 36, 38 und 36', 38' einer Seite I bzw. II nicht gleichzeitig, sondern nacheinander (seriell) angesteuert werden. Der Antrieb erfolgt nach dem Zahnstangenprinzip, wobei ein Antriebszahnrad 50, 65 in Richtung der Antriebsachse 51, 65 schrittweise von Blendplatte zu Blendplatte 36, 38 zur Verstellung weitergestellt wird. Man könnte dies als "mechanisches Multiplexing" bezeichnen. Während der Steuerung einer Blendplatte sind die übrigen Blendplatten durch die Verriegelungsverzahnung (Zahnstangen 54, 56, 62) in ihren Positionen fixiert. Der Vorteil des seriellen Ansteuerungsprinzips liegt gegenüber Vielblattkollimatoren mit paralleler Ansteuerung in der wesentlich einfacheren Konstruktion, die geringeren Platz beansprucht und aufgrund des geringeren Gewichts auch als Nachrüstung an vorhandene Bestrahlungsgeräte eingesetzt werden kann. Auch asymmetrische Bestrahlungsfelder lassen sich relativ rasch und feinstufig einstellen.

Der Applikationsbereich irregulärer Bestrahlungsfelder betrifft praktisch nur Bestrahlungstechniken mit festen Einstrahlungsrichtungen. Hierbei spielt der Zeitbedarf von einigen Sekunden zur motorischen Profileinstellung keine Rolle. Darüber hinaus hat sich gezeigt, daß die mit kontinuierlichen Bewegungsstrahlungen erzielten Dosisverteilungen auch durch Bestrahlungen aus vielen festen Einstrahlungsrichtungen erreicht werden können. Dies ist mit dem vorliegenden Vielblattkollimator mit serieller Ansteuerung ohne weiteres möglich, so daß dieser Vielblattkollimator im gesamten Spektrum der strahlentherapeutischen Bestrahlungstechniken eingesetzt werden kann.

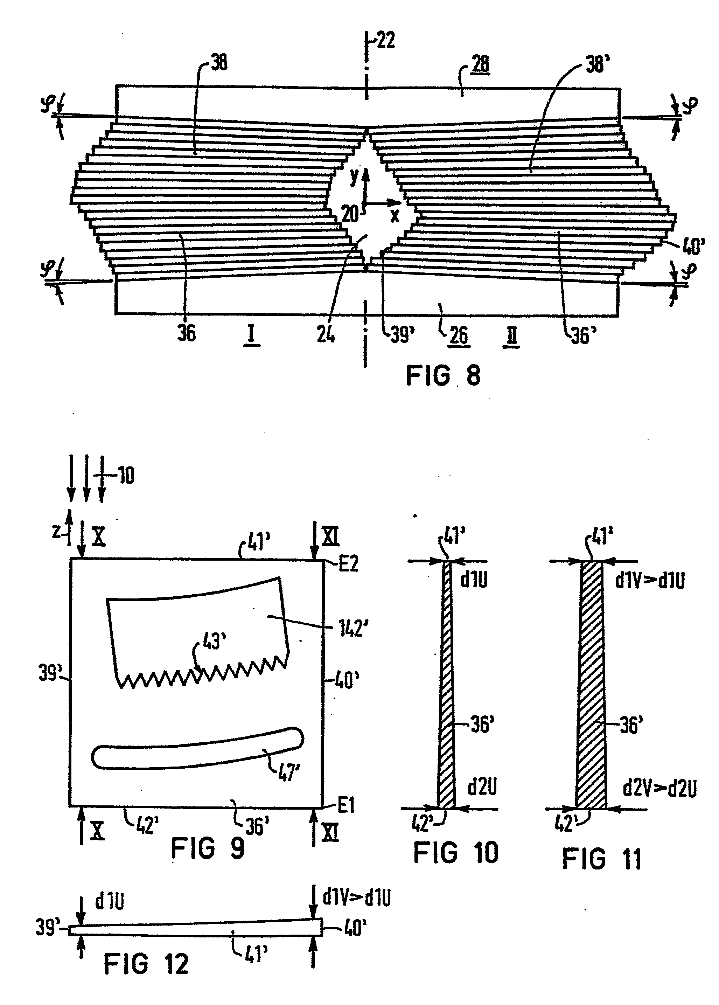

Nach Fig. 8 besteht der Konturenkollimator 4 wieder aus zwei Teilen oder Seiten I und II, die bezüglich einer vertikalen Ebene (y/z-Ebene) symmetrisch zueinander ausgebildet sind. Durch diese vertikale Ebene verläuft eine Symmetrielinie 20, die auf einer Mittellinie 22 senkrecht steht. Bei optimaler Justierung fällt die von einem Fokus ausgehende Hauptstrahlrichtung eines Strahlenbündels hochenergetischer Strahlung (z.B. Röntgenstrahlung) mit der Symmetrielinie zusammen. Die im Bereich der Symmetrielinie 20 liegende, durch Ausblendung erzielte Strahlenkontur (Profil) ist wieder mit 24 bezeichnet.

In einem gewissen Abstand voneinander sind eine erste und eine zweite vertikal gestellte, seitliche Außenplatte 26 bzw. 28 mit den Außenflächen parallel zueinander angeordnet. Das Besondere dieser Außenplatten 26, 28 besteht darin, daß ihre Innenflächen, und zwar ausgehend von der Mittellinie 22, jeweils unter einem vorgegebenen (kleinen) Winkel Phi zur Normalen (x-Richtung) dieser Mittellinie 22 ausgerichtet sind. Vorliegend ist dabei folgende Konstruktion gewählt: Ausgehend von der Mittellinie 22 verjüngen sich die beiden Außenplatten 26, 28 nach außen hin, d.h. in ± x-Richtung. Diese Verjüngung ist der Übersichtlichkeit wegen in Fig. 8 übertrieben dargestellt. Die Verjüngung ist in Wirklichkeit relativ gering, für die Funktion aber bedeutsam. Die beidseitig von der Mittellinie 22 einsetzende Verjüngung beträgt beispielsweise nur 0,54 mm (gemessen in y-Richtung) bei Fortschreiten um 10 cm nach außen (in +x- oder -x-Richtung). Das ergibt Phi gleich $5,4 \times 10^{-3}$.

Der bei Fortschreiten in x-Richtung sich vergrößernde Abstand der beiden Innenflächen der Außenplatten 26, 28 gewährleistet, daß ein Klemmen beim Auffahren oder Öffnen der im folgenden beschriebenen Blendplatten 26, 38, 36', 38' nicht stattfinden kann.

Zwischen den beiden seitlichen Außenplatten 26, 28 ist wieder sowohl links als auch rechts ein Paket aus zwei Gruppen von gegeneinander verschieblichen Blendplatten 36, 38 bzw. 36', 38' angeordnet. Alle Blendplatten 36, 38, 36', 38' in der vorderen bzw. hinteren Gruppe sind auf beiden Seiten I und II in gleicher Weise aufgebaut und nebeneinanderliegend angeordnet. Das Paket 36, 38 kann ebenso wie das Paket 36', 38' beispielsweise 27 Blendplatten umfassen. Die Blendplatten 36, 38, 36', 38' bestehen aus einem gut verarbeitbaren Material, sind strahlungsresistent und in Strahlungsrichtung z gesehen strahlungsundurchlässig. Jede der Blendplatten kann beispielsweise eine Fläche von 10 cm x 10 cm aufweisen. Als Material kommt bevorzugt eine Wolfram-Nickel-Legierung zur Anwendung.

Aus Fig. 9 ist die Seitenansicht einer der Blendplatten 36' zu entnehmen. Diese ist quadratisch ausgebildet und besitzt eine gekrümmte Führungsrille 47', die zum Schwenken um den Fokus der Strahlenquelle dient. Eine im oberen Teil der Blendplatte 36' untergebrachte Ausnehmung 142' ist am unteren Rand mit einer Verzahnung 43' versehen, die mit einem (nicht gezeigten) Stellorgan zum feinfühligen Schwenken oder Verschieben der Blendplatte 36' im Eingriff steht.

Es war bereits erwähnt worden, daß die Innenflächen der Außenplatten 26, 28 nach außen (in x-Richtung) voneinander zurückweichen. Damit kann, wie ebenfalls bereits dargelegt, ein Klemmen der Blendplatten 36, 38, 36', 38' beim Herausfahren verhindert werden. Würden nun aber planparallele Blendplatten verwendet werden, so könnte sich beim Rück- oder Zufahren in Richtung in Richtung auf die Mittellinie 22 ein Wackeln der Blendplatten ergeben. Damit würden zwischen den Blendplatten Zwischenräume geschaffen, durch die Strahlung hindurchtreten könnte. Dies muß aber unter allen Umständen verhindert werden. Um dieses zu gewährleisten, sind alle Blendplatten 36, 38, 36', 38' in Richtung auf die Mittellinie 22 leicht keilförmig geschliffen. Dies wird im folgenden anhand der Fig. 9 bis 12 verdeutlicht.

Aus den Fig. 9 bis 12 ergibt sich, daß die Blendplatte 36' an der der Mittellinie 22 der Strahlung 10 zugewandten Kante 39' eine Dicke d1U, d2U (vgl. Schnitt X-X durch den linken Teil der Blendplatte 36') und an der von der Mittellinie 22 der Strahlung 10 abgewandten Kante 40' eine Dicke d1V, d2V (vgl. Schnitt XI-XI durch den rechten Teil der Blendplatte 36') aufweist. Es gilt, daß d1V größer ist als d1U; ebenso ist d2V größer als d2U. Dies wird noch einmal bei einem Blick auf Fig. 12, d.h. auf die der Strahlung zugewandte Kante 41' der Blendplatte 36', deutlich.

Der Keilwinkel, der sich beim Fortschreiten von der Kante 39' zur Kante 40' ergibt, liegt im Bereich von 1/100 mm oder etwas mehr, insbesondere bei 4/100 mm, bei einer Blendplatte 36' einer Breite von 10 cm. Eine ausreichende Maßgenauigkeit kann bei der Bearbeitung eines Materials wie der voranstehend erwähnten Wolfram-Nickel-Legierung gewährleistet werden.

Als zusätzliche Maßnahme ist vorgesehen, daß die Blendplatten 36, 38, 36', 38' an der der Strahlung 10 zugewandten Kante 41' eine Dicke d1U, d1V aufweisen, die kleiner ist als die Dicke d2U, d2V an der von der Strahlung 10 abgewandten Kante 42'. Man kann diese beiden Tatsachen auch so ausdrücken: Die Blendplatte 36' ist in zwei aufeinander senkrechten Richtungen, die vorgegeben sind durch die Kanten 41', 42' und 39', 40', keilförmig geschliffen.

Zusammenfassend läßt sich also folgendes sagen: Aus fertigungstechnischen Gründen können Blendplatten häufig nicht ideal in Richtung des Fokus verjüngt geschliffen werden. Um den geometrischen Fehler möglichst gering zu halten, werden die Blendplatten 36, 38, 36', 38' (bezogen auf den Radius der Führungsrille 47) vorliegend zum Radiusmittelpunkt hin verjüngt geschliffen.

Man kann dies auch so ausdrücken: Da die Blendplatten 36, 38, 36', 38' mit ihrer von der Mittellinie 22 abgewandten Seite (z.B. 40' in Fig. 9) während des Verfahrens in der Führungsrille in x-Richtung zum Fokus einen Radius beschreiben, steigt z.B. die untere Ecke E1 (vgl. Fig. 9) der verfahrenden, nach oben sich verjüngenden Blendplatten 36' gegenüber den restlichen Blendplatten 36' und den feststehenden Außenplatten 26, 28 nach oben. Wenn nicht genügend Spiel vorhanden ist - was wegen der Gefahr des Strahlendurchsatzes unerwünscht ist - kann es bei den früher beschriebenen Blendplatten zur Klemmung kommen. Um dies zu vermeiden, sind vorliegend die Außenplatten 26, 28 um die Dickendifferenz der einzelnen, nach oben ansteigenden Blendplatten 36, 38, 36', 38', multipliziert mit der Anzahl der Blendplatten 36, 38, 36' bzw. 38', nach außen freigeschliffen (kleiner Winkel Phi, vgl. Fig. 8).

**Patentansprüche**

1. Konturenkollimator für die Strahlentherapie mit einer vorgegebenen Anzahl von gegeneinander verschieblich angeordneten Blendplatten, **gekennzeichnet durch**
   a) eine Verzahnung (43) an jeder Blendplatte (36),
   b) ein der vorgegebenen Anzahl von Blendplatten (36) gemeinsames Verstellorgan (50, 51, 52) zum Verschieben jeweils einer ersten der Blendplatten (36) gegenüber den restlichen Blendplatten (36), welches Verstellorgan (50, 51, 52) mit der Verzahnung (43) der ersten Blendplatte (36) im Eingriff steht,
   c) eine mit den Verzahnungen (43) der restlichen Blendplatten (36) im Eingriff stehende Verriegelungseinrichtung (54, 56) und
   d) eine Einrichtung (71 bis 76) zum Verschieben des Verstellorgans (50, 51, 52) von der Verzahnung (43) der ersten Blendplatte (36) zur Verzahnung (43) einer weiteren zweiten Blendplatte (36), bei welchem Verschieben die erste Blendplatte (36) verriegelt und die zweite Blendplatte (36) entriegelt wird.

2. Konturenkollimator nach Anspruch 1, **gekennzeichnet durch**
   a eine weitere vorgegebene Anzahl von Blendplatten (38), von denen jede mit einer Verzahnung (43) versehen ist, neben der vorgegebenen Anzahl von Blendplatten (36),
   b) ein der weiteren vorgegebenen Anzahl von Blendplatten (38) gemeinsames weiteres Verstellorgan (64, 65, 66) zum Verschieben jeweils einer ersten weiteren Blendplatte (38) gegenüber den restlichen weiteren Blendplatten (38), welches weitere Verstellorgan (64, 65, 66) mit der Verzahnung (43) der ersten weiteren Blendplatte (38) im Eingriff steht,
   c) eine mit den Verzahnungen (43) der restlichen weiteren Blendplatten (38) im Eingriff stehende weitere Verriegelungseinrichtung (56, 62) und
   d) eine Einrichtung (71 bis 76) zum Verschieben des weiteren Verstellorgans (64, 65, 66) von der Verzahnung (43) der ersten weiteren Blendplatte (38) zur Verzahnung (43) einer benachbarten zweiten weiteren Blendplatte (38), bei welchem Verschieben die erste weitere Blendplatte (38) verriegelt und die zweite weitere Blendplatte (38) entriegelt wird.

3. Konturenkollimator nach Anspruch 2, **dadurch gekennzeichnet,** daß die Einrichtung (71 bis 76) zum Verschieben des weiteren Verstellorgans (64, 65, 66) durch die Einrichtung (71 bis 76) des erstgenannten Verstellorgans (50, 51, 52) gebildet ist.

4. Konturenkollimator nach einem der Ansprüche 1 bis 3, **dadurch gekennzeichnet,** daß er bezüglich einer Mittellinie (22) symmetrisch aufgebaut ist und eine Anzahl gegeneinander verschieblich angeordneter zusätzlicher Blendplatten (36') aufweist, wobei die erstgenannten Blendplatten (36) und die zusätzlichen Blendplatten (36') aufeinander zu beweglich sind.

5. Konturenkollimator nach einem der Ansprüche 1 bis 4, **dadurch gekennzeichnet,** daß die Verzahnung (43) an einem Rand der Blendplatten (36, 38, 36', 38'; 37) angeordnet ist.

6. Konturenkollimator nach einem der Ansprüche 1 bis 5, **dadurch gekennzeichnet,** daß die Blendplatten (36, 38, 36', 38'; 37) um einen gemeinsamen Fokus (F) schwenkbar sind.

7. Konturenkollimator nach Anspruch 6, **dadurch gekennzeichnet,** daß die Blendplatten (37') jeweils mit einer Führungsrille (47') versehen sind.

8. Konturenkollimator nach Anspruch 7, **dadurch gekennzeichnet,** daß die Führungsrille (47') gekrümmt ist.

9. Konturenkollimator nach Anspruch 7 oder 8, **dadurch gekennzeichnet,** daß zur Führung der Blendplatten (37') mittels der Führungsrillen (47') zwei parallel zueinander angeordnete Führungsbolzen (48, 49, 48', 49') vorgesehen sind.

10. Konturenkollimator nach einem der Ansprüche 1 bis 9, **dadurch gekennzeichnet,** daß das Verstellorgan (50 bis 52, 64 bis 66) ein Antriebszahnrad (50, 64) umfaßt, das etwa die Dicke (d1) einer Blendplatte (36, 38, 36', 38'; 37) besitzt.

11. Konturenkollimator nach Anspruch 10, **dadurch gekennzeichnet,** daß das Antriebszahnrad (50, 64) über eine Einstellwelle (51, 65) mit einem Motor (52, 66) verbunden ist.

12. Konturenkollimator nach Anspruch 11, **dadurch gekennzeichnet,** daß das Antriebszahnrad (50, 64) im Bereich des einen Endes der Einstellwelle (51, 65) befestigt ist.

13. Konturenkollimator nach einem der Ansprüche 1 bis 12, **dadurch gekennzeichnet,** daß die Verriegelungseinrichtung (54, 56; 56, 62) zumindest eine verzahnte Welle (54, 56, 62) umfaßt, die in die Verzahnungen (43) zumindest einiger Blendplatten (36, 38, 36', 38'; 37) eingreift.

14. Konturenkollimator nach Anspruch 11 oder 12 sowie nach Anspruch 13, **dadurch gekennzeichnet,** daß daß die Einstellwelle (51, 62) durch eine Längsbohrung in der verzahnten Welle (54, 56, 62) geführt ist.

15. Konturenkollimator nach Anspruch 2 und 14, **dadurch gekennzeichnet,** daß drei axial ausgerichtete verzahnte Wellen (54, 56, 62) mit Längsbohrungen und zwei dazwischen axial angeordnete Antriebsräder (50, 64) vorgesehen sind, wobei die Antriebsräder (50, 64) denselben Durchmesser, dieselbe Zähnezahl und dieselbe Zahnteilung aufweisen wie die verzahnten Wellen (54, 56, 62).

16. Konturenkollimator nach einem der Ansprüche 13 bis 15, **dadurch gekennzeichnet,** daß die Blendplatten (36, 38) zwischen zwei parallel zueinander ausgerichteten Außenplatten (26, 28) angeordnet sind, die vorzugsweise an einer Kante mit einer Verzahnung (30, 32) versehen sind, in die die verzahnte Welle (54, 62) eingreift.

17. Konturenkollimator nach einem der Ansprüche 1 bis 16, insbesondere nach Anspruch 13, **dadurch gekennzeichnet,** daß die Verriegelungseinrichtung (54, 56) mit der Einrichtung (71 bis 76) zum Verschieben des Verstellorgans (50 bis 52, 64 bis 66) gekoppelt ist.

18. Konturenkollimator nach einem der Ansprüche 1 bis 17, **dadurch gekennzeichnet,** daß die Einrichtung zum Verschieben des Verstellorgans (50 bis 52, 64 bis 66) ein Rahmengestell (60) umfaßt, das von einem Motor (75) in einer Richtung (y) quer zu den Blendplatten (36, 38) verschiebbar ist.

19. Konturenkollimator nach Anspruch 13 und 18, **dadurch gekennzeichnet,** daß die verzahnte Welle (54, 56; 62) zwischen zwei Seitenarmen (71, 72) des Rahmengestells (60) angeordnet ist.

20. Konturenkollimator nach Anspruch 18 oder 19, **dadurch gekennzeichnet,** daß der Motor (75) über eine Spindel (76) mit dem Rahmengestell (60) verbunden und die Spindel (76) in einem Gewinde, das in einem Halteblock (78) angeordnet ist, drehbar ist.

21. Konturenkollimator nach Anspruch 19, **dadurch gekennzeichnet,** daß mindestens zwei verzahnte Wellen (54, 56, 62) vorgesehen sind, zwischen denen ein Antriebszahnrad (50, 64) drehbar angeordnet ist, daß die beiden verzahnten Wellen (54, 56, 62) durch ein Verbindungsteil (57) miteinander verbunden sind, und daß die beiden verzahnten Wellen (54, 56, 62) durch eine Druckeinrichtung (57, 81, 83, 84, 87, 88) an die Blendplatten (36, 38) angedrückt sind.

22. Konturenkollimator nach Anspruch 21, **dadurch gekennzeichnet,** daß die Druckeinrichtung ein Andruckstück (81) umfaßt, das beim Verschieben des Rahmengestells (60) auf dem Verbindungsteil (57) gleitet.

23. Konturenkollimator nach Anspruch 21 oder 22, **dadurch gekennzeichnet,** daß die Druckeinrichtung einen Andruckbügel (83) aufweist, der an zwei beabstandeten Seitenbügeln (87, 88) befestigt ist, welche Seitenbügel (87, 88) ihrerseits zwischen sich die Blendplatten (36, 38) einschließen.

24. Konturenkollimator nach einem der Ansprüche 18 bis 23, **dadurch gekennzeichnet,** daß das Rahmengestell (60) auf einer Gleitachse (79) gleitend angeordnet ist.

25. Konturenkollimator nach einem der Ansprüche 1 bis 24, **dadurch gekennzeichnet,** daß die Blendplatten (36, 38, 36', 38'; 37) an der der Strahlung zugewandten Kante (41') eine kleinere Dicke (d1) aufweisen als an der von der Strahlung abgewandten Kante (42').

26. Konturenkollimator nach einem der Ansprüche 1 bis 25, **dadurch gekennzeichnet,** daß die Verzahnung (43') der Blendplatten (37') eine Reihe dreieckförmig geformter Zähne (45') aufweist.

27. Konturenkollimator nach einem der Ansprüche 1 bis 26, **dadurch gekennzeichnet,** daß die Blendplatten (36, 38, 36', 38'; 37) aus einem Wolfram enthaltenden Material bestehen.

28. Konturenkollimator nach einem der Ansprüche 4 bis 27, **dadurch gekennzeichnet,** daß die Blendplatten (36) und die zusätzlichen Blendplatten (38) jeweils über die Mittellinie (22) hinaus in die andere Seite (I, II) verschiebbar sind.

29. Konturenkollimator nach einem der Ansprüche 1 bis 28, **dadurch gekennzeichnet,** daß eine Einrichtung zur Rückmeldung der Verschiebestellung der einzelnen Blendplatten (37, 37') vorgesehen ist.

30. Konturenkollimator nach Anspruch 29, **dadurch gekennzeichnet,** daß die Einrichtung einen Zähler aufweist, der bei jedem Verschieben die Zahl der betätigten Zähne (45') der Verzahnung (37') der betreffenden Blendplatte (37') zählt.

31. Konturenkollimator nach einem der Ansprüche 1 bis 30, **dadurch gekennzeichnet,** daß die Blendplatten (36, 38, 36', 38') zwischen zwei Außenplatten (26, 28) angeordnet und mit einer Kante (39') an eine Mittellinie (22) heranschiebbar sind, durch die eine Symmetrielinie (20) der Strahlung (10) verläuft, daß die Innenflächen der Außenplatten (26, 28) unter vorgegebenem Winkel (Phi) zur Normalen (x) der Mittellinie (22) ausgerichtet sind, und daß die Blendplatten (26, 38, 36', 38') an der Mittellinie (22) der Strahlung (10) zugewandten Kante (39') eine geringere Dicke (d1U, d2U) aufweisen als an der von der Mittellinie (22) der Strahlung (10) abgewandten Kante (40') (Fig. 8 bis 12).

32. Konturenkollimator nach Anspruch 31, **dadurch gekennzeichnet,** daß die Blendplatten (36, 38, 36', 38') an der der Strahlung (10) zuegewandten Kante (41') eine kleinere Dicke (d1U, d1V) aufweisen als an der von der Strahlung (10) abgewandten Kante (42').

33. Konturenkollimator nach Anspruch 32, **dadurch gekennzeichnet,** daß die Blendplatten (36, 38, 36', 38') in zwei aufeinander senkrechten Richtungen keilförmig geschliffen sind.

34. Konturenkollimator nach einem der Ansprüche 31 bis 33, **dadurch gekennzeichnet,** daß die Außenplatten (26, 28) - von der Mittellinie (22) aus - sich nach außen (x-Richtung) verjüngen.

35. Konturenkollimator nach Anspruch 34, **dadurch gekennzeichnet,** daß sich bei einer Fokusentfernung der Strahlquelle von den oberen Kanten (41') der Blendplatten (36, 38, 36', 38') von etwa 46 cm die Außenplatten (26, 28) auf jeweils 10 cm etwa 0,54 mm verjüngen.

36. Konturenkollimator nach Anspruch 34 oder 35, **dadurch gekennzeichnet,** daß die Außenplatten (26, 28) sich beidseitig von der Mittellinie (22) aus nach außen verjüngen.

**Claims**

1. Contour collimator for radiation therapy having a specified number of diaphragm plates arranged so that they can be displaced relative to one another, characterized by

    a) a toothed arrangement (43) at each diaphragm plate (36),

    b) an adjusting part (50, 51, 52) common to the specified number of diaphragm plates (36) for displacing in each case a first one of the diaphragm plates (36) relative to the remaining diaphragm plates (36), which adjusting part (50, 51, 52) is engaged with the teeth arrangement (43) of the first diaphragm plate (36),

    c) a locking device (54, 56) engaged with the toothed arrangements (43) of the remaining diaphragm plates (36) and

    d) a device (71 to 76) for displacing the adjusting part (50, 51, 52) from the toothed arrangement (43) of the first diaphragm plate (36) to the toothed arrangement (43) of a further second diaphragm plate (36), with which displacement the first diaphragm plate (36) is locked and the second diaphragm plate (36) is unlocked.

2. Contour collimator according to claim 1, characterized by
a) a further specified number of diaphragm plates (38), each of which is provided with a toothed arrangement (43), in addition to the specified number of diaphragm plates (36),
b) a further adjusting part (64, 65, 66) common to the further specified number of diaphragm plates (38) for displacing in each case a first further diaphragm plate (38) relative to the remaining further. diaphragm plates (38), which further adjusting part (64, 65, 66) is engaged with the toothed arrangement (43) of the first further diaphragm plate (38),
c) a further locking device (56, 62) engaged with the toothed arrangements (43) of the remaining further diaphragm plates (38) and,
d) a device (71 to 76) for displacing the further adjusting part (64, 65, 66) from the toothed arrangement (43) of the first further diaphragm plate (38) to the toothed arrangement (43) of an adjacent second further diaphragm plate (38), with which displacement the first further diaphragm plate (38) is locked and the second further diaphragm plate (38) is unlocked.

3. Contour collimator according to claim 2, characterized in that the device (71 to 76) for displacing the further adjusting part (64, 65, 66) is formed by the device (71 to 76) of the first-named adjusting part (50, 51, 52).

4. Contour collimator according to one of claims 1 to 3, characterized in that it is constructed symmetrical with regard to a centre line (22) and has a number of additional diaphragm plates (36') arranged so that they can be displaced relative to one another, whereby the first-named diaphragm plates (36) and the additional diaphragm plates (36') are moveable towards one another.

5. Contour collimator according to one of claims 1 to 4, characterized in that the toothed arrangement (43) is arranged at one edge of the diaphragm plates (36, 38, 36', 38'; 37).

6. Contour collimator according to one of claims 1 to 5, characterized in that the diaphragm plates (36, 38, 36', 38'; 37) can be rotated around a common focus (F).

7. Contour collimator according to claim 6, characterized in that the diaphragm plates (37') in each case are provided with a guiding groove (47').

8. Contour collimator according to claim 7, characterized in that the guiding groove (47') is curved.

9. Contour collimator according to claim 7 or 8, characterized in that to guide the diaphragm plates (37') by means of the guiding grooves (47') two guiding pins (48, 49, 48', 49') are provided arranged parallel to one another.

10. Contour collimator according to one of claims 1 to 9, characterized in that the adjusting part (50 to 52, 64 to 66) comprises a drive gear wheel (50, 64) which has approximately the thickness (d1) of a diaphragm plate (36, 38, 36', 38'; 37).

11. Contour collimator according to claim 10, characterized in that the drive gear wheel (50, 64) is connected by means of an adjusting shaft (51, 65) to a motor (52, 66).

12. Contour collimator according to claim 11, characterized in that the drive gear wheel (50, 64) is fastened in the region of one end of the adjusting shaft (51, 65).

13. Contour collimator according to one of claims 1 to 12, characterized in that the locking device (54, 56; 56, 62) comprises at least one toothed shaft (54, 56, 62) which engages into the toothed arrangements (43) of at least a few diaphragm plates (36, 38, 36', 38'; 37).

14. Contour collimator according to claim 11 or 12 and according to claim 13, characterized in that the adjusting shaft (51, 62) is guided through a longitudinal borehole in the toothed shaft (54, 56, 62).

15. Contour collimator according to claim 2 and 14, characterized in that three axially aligned toothed shafts (54, 56, 62), with longitudinal boreholes, and two drive wheels (50, 64), arranged axially between them, are provided, whereby the drive gear wheels (50, 64) have the same diameter, the same number of teeth and the same tooth spacing as the toothed shafts (54, 56, 62).

16. Contour collimator according to one of claims 13 to 15, characterized in that the diaphragm plates (36, 38) are arranged between two outer plates (26, 28) aligned parallel to one another, which are provided with a toothed arrangement (30, 32) preferably on an edge, into which toothed arrangement the toothed shaft (54, 62) engages.

17. Contour collimator according to one of claims 1 to 16, more particularly according to claim 13, characterized in that the locking device (54, 56) is coupled with the device (71 to 76) for displacing the adjusting part (50 to 52, 64 to 66).

18. Contour collimator according to one of claims 1 to 17, characterized in that the device for displacing the adjusting part (50 to 52, 64 to 66) comprises a frame (60) which can be displaced by a motor (75) in a direction (y) transverse to the diaphragm plates (36, 38).

19. Contour collimator according to claim 13 and 18, characterized in that the toothed shaft (54, 56; 62) is arranged between two side arms (71, 72) of the frame (60).

20. Contour collimator according to claim 18 or 19, characterized in that the motor (75) is connected by means of a spindle (76) to the frame (60) and the spindle (76) can be rotated in a thread which is arranged in a holding block (78).

21. Contour collimator according to claim 19, characterized in that at least two toothed shafts (54, 56, 62) are provided, between which a drive gear wheel (50, 64) is rotatably arranged, in that the two toothed shafts (54, 56, 62) are connected to one another by a connecting part (57), and in that the two toothed shafts (54, 56, 62) are pressed onto the diaphragm plates (36, 38) by means of a pressure device (57, 81, 83, 84, 87, 88).

22. Contour collimator according to claim 21, characterized in that the pressure device comprises a pressing piece (81) which slides on the connecting part (57) when the frame (60) is displaced.

23. Contour collimator according to claim 21 or 22, characterized in that the pressure device has a pressure strap (83) which is fastened to two spaced apart side straps (87, 88), which side straps (87, 88) for their part enclose between them the diaphragm plates (36, 38).

24. Contour collimator according to one of claims 18 to 23, characterized in that the frame (60) is arranged in a sliding manner on a sliding axis (79).

25. Contour collimator according to one of claims 1 to 24, characterized in that the diaphragm plates (36, 38, 36', 38'; 37) have on the edge (41') facing the radiation a smaller thickness (d1) than on the edge (42') directed away from the radiation.

26. Contour collimator according to one of claims 1 to 25, characterized in that the toothed arrangement (43') of the diaphragm plates (37') has a series of teeth (45') formed in a triangular manner.

27. Contour collimator according to one of claims 1 to 26, characterized in that the diaphragm plates (36, 38, 36', 38'; 37) consist of a material containing tungsten.

28. Contour collimator according to one of claims 4 to 27, characterized in that the diaphragm plates (36) and the additional diaphragm plates (38) in each case can be displaced via the centre line (22) out into the other side (I, II).

29. Contour collimator according to one of claims 1 to 28, characterized in that a device for remote indication of the displacement position of the individual diaphragm plates (37, 37') is provided.

30. Contour collimator according to claim 29, characterized in that the device has a counter which at every displacement counts the number of the moving teeth (45') of the toothed arrangement (37') of the diaphragm plate (37') concerned.

31. Contour collimator according to one of claims 1 to 30, characterized in that the diaphragm plates (36, 38, 36', 38') are arranged between two outer plates (26, 28) and can be shifted forward with an edge (39') on a centre line (22), through which a symmetry line (20) of the radiation (10) extends, in that the inner surfaces of the outer plates (26, 28) are aligned at a specified angle (Phi) to the normal (X) of the centre line (22), and in that the diaphragm plates (26, 38, 36', 38') on the edge (39') facing the centre line (22) of the radiation (10) have a smaller thickness (d1U, d2U) than on the edge (40') directed away from the centre line (22) of the radiation (10) (Figures 8 to 12).

32. Contour collimator according to claim 31, characterized in that the diaphragm plates (36, 38, 36', 38') on the edge (41') facing the radiation (10) have a smaller thickness (d1U, d1V) than on the edge (42') directed away from the radiation (10).

33. Contour collimator according to claim 32, characterized in that the diaphragm plates (36, 38, 36', 38') are ground in a wedge-shaped manner in two directions perpendicular to one another.

34. Contour collimator according to one of claims 31 to 33, characterized in that the outer plates (26, 28) - from the centre line (22) - taper to the outside (x-direction).

35. Contour collimator according to claim 34, characterized in that with a focussing distance of the radiation source from the upper edges (41') of the diaphragm plates (36, 38, 36', 38') from about 46 cm, the outer plates (26, 28) taper to about 0.54 mm for each 10 cm.

36. Contour collimator according to claim 34 or 35, characterized in that the outer plates (26, 28) taper to the outside on both sides from the centre line (22).

**Revendications**

1. Collimateur de contour pour la radiothérapie, comportant un nombre prédéterminé de plaques collimatrices mobiles les unes par rapport aux autres, caractérisé par

a) une denture (43) prévue sur chaque plaque collimatrice (36),

b) un organe de réglage (50, 51, 52), commun au nombre prédéterminé de plaques collimatrices (36) et servant à déplacer respectivement une première des plaques collimatrices (36) par rapport aux autres plaques collimatrices (36), cet organe de réglage (50, 51, 52) engrenant avec la denture (43) de la première plaque collimatrice (36),

c) un dispositif de verrouillage (54, 56) en prise avec les dentures (43) des autres plaques collimatrices (36), et

d) un dispositif (71 à 76) servant à déplacer l'organe de réglage (50, 51, 52) depuis la denture (43) de la première plaque collimatrice (36) jusqu'à la denture (43) du seconde autre plaque collimatrice (36), déplacement lors duquel la première plaque collimatrice (36) est verrouillé et la seconde plaque collimatrice (36) est déverrouillée.

2. Collimateur de contour suivant la revendication 1, caractérisé par

a) un autre nombre prédéterminé de plaques collimatrices (38), dont chacune comporte une denture (43), en plus du nombre prédéterminé de plaques collimatrices (36),

b) un autre organe de réglage (64, 65, 66) commun audit autre nombre prédéminé de plaques collimatrices (38) pour déplacer respectivement une première autre plaque collimatrice (36) par rapport aux autres plaques collimatrices restantes (38), lequel autre organe de réglage (64, 65, 66) engrène avec la denture (43) de la première autre plaque collimatrice (38),

c) un autre dispositif de verrouillage (56, 62) engrenant avec les dentures (43) des autres plaques collimatrices restantes (38), et

d) un dispositif (71 à 76) servant à déplacer l'autre organe de réglage (64, 65, 66) depuis la denture (43) de la première autre plaque collimatrice (38) jusqu'à la denture (43) d'une seconde plaque collimatrice voisine (38), déplacement lors duquel la première autre plaque collimatrice (38) est verrouillée et la seconde autre plaque collimatrice (38) est déverrouillée.

3. Collimateur de contour suivant la revendication 1, caractérisé par le fait que le dispositif (71 à 76) est agencé de manière à déplacer l'autre organe de réglage (64, 65, 66) au moyen du dispositif (71 à 76) de l'organe de réglage (50, 51, 52) indiqué en premier.

4. Collimateur de contour suivant l'une des revendications 1 à 3, caractérisé par le fait qu'il est agencé symétriquement par rapport à un axe central (22) et possède un nombre de plaques collimatrices supplémentaires (36') déplaçables les unes par rapport aux autres, les plaques collimatrices (36) indiquées en premier lieu et les plaques collimatrices supplémentaires (36') étant déplaçables les unes sur les autres.

5. Collimateur de contour suivant l'une des revendications 1 à 4, caractérisé par le fait que la denture (43) est disposée sur un bord des plaques collimatrices (36, 38, 36', 38'; 37).

6. Collimateur de contour suivant l'une des revendications 1 à 5, caractérisé par le fait que les plaques collimatrices (36, 38, 36', 38'; 37) peuvent pivoter autour d'un foyer commun (F).

7. Collimateur de contour suivant la revendication 6, caractérisé par le fait que les plaques collimatrices (37') comportent chacune une rainure de guidage (47').

8. Collimateur de contour suivant la revendication 7, caractérisé par le fait que la rainure de guidage (47') est courbe.

9. Collimateur de contour suivant la revendication 7 ou 8, caractérisé par le fait que pour le guidage des plaques collimatrices (37') au moyen des rainures de guidage (47'), il est prévu deux tétons de guidage (48, 49, 48', 49') parallèles l'un à l'autre.

10. Collimateur de contour suivant l'une des revendications 1 à 9, caractérisé par le fait que l'organe de réglage (50 à 52, 64 à 66) comporte un pignon d'entraînement (50, 75), qui possède approximativement l'épaisseur (d1) d'une plaque collimatrice (36, 38, 36', 38'; 37).

11. Collimateur de contour suivant la revendication 10, caractérisé par le fait que le pignon d'entraînement (50, 64) est relié à un moteur (52, 66) par l'intermédiaire d'un arbre de réglage (51, 65).

12. Collimateur de contour suivant la revendication 11, caractérisé par le fait que le pignon d'entraînement (50, 64) est fixé au voisinage des extrémités de l'arbre de réglage (51, 65).

13. Collimateur de contour suivant l'une des revendications 1 à 12, caractérisé par le fait que le dispositif de verrouillage (54, 56; 56, 62) comporte au moins un arbre denté (54, 56, 62), qui engrène dans les dentures (43) d'au moins quelques plaques collimatrices (36, 38, 36', 38'; 37).

14. Collimateur de contour suivant la revendication 11 ou 12, ainsi que la revendication 13, caractérisé par le fait que l'arbre de réglage (51, 62) est guidé dans un perçage longitudinal ménagé dans l'arbre denté (54, 56, 62).

15. Collimateur de contour suivant les revendications 2 et 14, caractérisé par le fait qu'il est prévu trois arbres dentés (54, 56, 62) alignés axialement, qui comportent des perçages longitudinaux, et deux pignons d'entraînement (50, 64) disposés axialement entre ces arbres, les pignons d'entraînement (50, 64) possédant le même diamètre, le même nombre de dents et le même pas de dents que les arbres dentés (54, 56, 62).

16. Collimateur de contour suivant l'une des revendications 13 à 15, caractérisé par le fait que les plaques collimatrices (36, 38) sont disposées entre deux plaques extérieures (26, 28) parallèles entre elles, qui comportent, de préférence au niveau d'un bord, une denture (30, 32), dans laquelle s'engage l'arbre denté (54, 62).

17. Collimateur de contour suivant l'une des revendications 1 à 16, notamment la revendication 13, caractérisé par le fait que le dispositif de verrouillage (54, 56) est accouplé au dispositif (71 à 76) pour déplacer l'organe de réglage (50 à 52, 64 à 66).

18. Collimateur de contour suivant l'une des revendications 1 à 17, caractérisé par le fait que le dispositif servant à déplacer l'organe de réglage (50 à 52, 64 à 68) comporte un châssis en forme de cadre (60), qui peut être déplacé par un moteur (75) dans une direction (y) transversalement par rapport aux plaques collimatrices (36, 38).

19. Collimateur des contour suivant les revendications 13 à 18, caractérisé par le fait que l'arbre d'entrée (54, 56; 62) est disposé entre deux bras latéraux (71, 72) du châssis en forme de cadre (60).

20. Collimateur de contour suivant la revendication 18 ou 19, caractérisé par le fait que le moteur (75) est relié par l'intermédiaire d'une broche (76) au châssis en forme de cadre (60) et que la broche (76) peut être entraînée en rotation dans un taraudage, qui est ménagé dans un bloc de retenu (78).

21. Collimateur de contour suivant la revendication 19, caractérisé par le fait qu'il est prévu au moins deux arbres dentés (54, 56, 62), entre lesquels un pignon d'entraînement (50, 54) est disposé de façon à pouvoir tourner, les deux arbres dentés (54, 56, 62) sont reliés entre eux par un élément de liaison (57) et que les deux arbres dentés (54, 56, 62) sont repoussés contre les plaques collimatrices (34, 36) par un dispositif de pression (57, 81, 83, 84, 87, 89).

22. Collimateur de contour suivant la revendication 21, caractérisé par le fait que le dispositif de pression comporte une pièce de serrage (81), qui glisse sur l'élément de liaison (57) lors du déplacement de châssis en forme de cadre (60).

23. Collimateur de contour suivant la revendication 21 ou 22, caractérisé par le fait que le dispositif de pression comporte un étrier de serrage (83) qui est fixé à deux étriers latéraux (87, 88) distants l'un de l'autre, qui, pour leur part, enserrent entre eux les plaques collimatrices (36, 38).

24. Collimateur de contour suivant l'une des revendications 18 à 23, caractérisé par le fait que le châssis en forme de cadre (60) est disposé de manière à glisser sur un axe de glissement (79).

25. Collimateur de contour suivant l'une des revendications 1 à 24, caractérisé par le fait que les plaques collimatrices (36, 38, 36', 38'; 37) possèdent, au niveau du bord (41') tourné vers le rayonnement, une épaisseur (d1) inférieure à celle qu'elles possèdent au niveau du bord (42') tourné à l'opposé du rayonnement.

26. Collimateur de contour suivant l'une des revendications 1 à 25, caractérisé par le fait que la denture (43′) des plaques collimatrices (37′) possède une série de dents (35′) de forme triangulaire.

27. Collimateur de contour suivant l'une des revendications 1 à 26, caractérisé par le fait que les plaques collimatrices (36, 38, 36′, 38′; 37) sont constituées par un matériau contenant du tungstène.

28. Collimateur de contour suivant l'une des revendications 1 à 27, caractérisé par le fait que les plaques collimatrices (36) et les plaques collimatrices supplémentaires (38) sont déplaçables respectivement au-delà de l'axe central (22) en direction de l'autre côté (I, II).

29. Collimateur de contour suivant l'une des revendications 1 à 28, caractérisé par le fait qu'il est prévu un dispositif pour signaler en retour la position de décalage des différentes plaques collimatrices (37, 37′).

30. Collimateur de contour suivant l'une des revendications 1 à 29, caractérisé par le fait que le dispositif comporte un compteur qui, lors de chaque déplacement, compte le nombre des dents actionnées (45′) de la denture (37′) de la plaque collimatrice (37′) considérée.

31. Collimateur de contour suivant l'une des revendications 1 à 30, caractérisé par le fait que les plaques de diaphragme (36, 38, 36′, 38′) sont disposées entre deux plaques extérieures (26, 28) et qu'un (39′) de leurs bords peut être rapproché d'un axe central (22) que recoupe un axe de symétrie (30) du rayonnement (10), que les surfaces intérieures des plaques extérieures (26, 28) sont alignées sous un angle prédéterminé (phi) par rapport à la normale (x) à l'axe central (22), et que les plaques collimatrices (36, 38, 36′, 38′) possèdent, au niveau de leur bord (39′) tourné vers l'axe central (22) du rayonnement (10), une épaisseur (d1U, d2U) plus faible qu'au niveau de leur bord (40′) tourné à l'opposé de l'axe central (22) du rayonnement (10) (figures 8 à 12).

32. Collimateur de contour selon la revendication 31, caractérisé en ce que les plaques collimatrices (36, 38, 36′, 38′) possèdent, au niveau de leur bord (41′) tourné vers le rayonnement (10), une épaisseur (d1U, d1V) plus faible qu'au niveau de leur bord (42′) tourné à l'opposé du rayonnement (10).

33. Collimateur de contour suivant la revendication 32, caractérisé par le fait que les plaques collimatrices (36, 38, 36′, 38′) sont meulées avec une forme en coin dans deux directions perpendiculaires entre elles.

34. Collimateur de contour suivant l'une des revendications 31 à 33, caractérisé par le fait que les plaques extérieures (26, 28) se rétrécissent vers l'extérieur (direction x) − à partir de l'axe central (22).

35. Collimateur de contour suivant la revendication 34, caractérisé par le fait que dans le cas où le foyer de la source de rayonnement est éloigné du bord supérieur (41′) des plaques collimatrices (36, 38, 36′, 38′) d'une distance à environ 46 cm, les plaques extérieures (26, 28) se rétrécissent d'environ 0,54 mm pour chaque longueur d'éloignement de 10 cm.

36. Collimateur de contour suivant la revendication 34 ou 35, caractérisé par le fait que les plaques extérieures (26, 28) se rétrécissent vers l'extérieur, des deux côtés, à partir de l'axe central (22).

FIG 1

FIG 2

EP 0 245 768 B1

FIG 3

FIG 4

FIG 6

FIG 5

FIG 7

EP 0 245 768 B1

**FIG 8**

**FIG 9**

**FIG 10**

**FIG 11**

**FIG 12**